(19) Europäisches Patentamt / European Patent Office / Office européen des brevets

(11) EP 1 289 529 B1

(12) EUROPÄISCHE PATENTSCHRIFT

(45) Veröffentlichungstag und Bekanntmachung des Hinweises auf die Patenterteilung:
**21.03.2007 Patentblatt 2007/12**

(21) Anmeldenummer: **01945112.9**

(22) Anmeldetag: **10.05.2001**

(51) Int Cl.:
***A61K 31/485*** *(2006.01)* ***A61K 38/06*** *(2006.01)*
***A61P 25/04*** *(2006.01)*

(86) Internationale Anmeldenummer:
**PCT/EP2001/005346**

(87) Internationale Veröffentlichungsnummer:
**WO 2001/091755 (06.12.2001 Gazette 2001/49)**

(54) **WIRKSTOFFKOMBINATION ENTHALTEND EINE VERBINDUNG MIT OPIOIDER WIRKSAMKEIT UND EINE WEITERE VERBINDUNG DER FORMEL I**

ACTIVE SUBSTANCE COMBINATION CONTAINING A COMPOUND WITH AN OPIOID EFFECT AND AT LEAST ONE FURTHER COMPOUND OF FORMULA I

COMBINAISON DE SUBSTANCES COMPRENANT UN COMPOSE A EFFET OPIOIDE ET UN AUTRE COMPOSE DE LA FORMULE I

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **26.05.2000 DE 10025948**

(43) Veröffentlichungstag der Anmeldung:
**12.03.2003 Patentblatt 2003/11**

(73) Patentinhaber: **Grünenthal GmbH**
**52078 Aachen (DE)**

(72) Erfinder:
- **CHIZH, Boris**
  **Whittlesford, Cambridge CB2 4NW (GB)**
- **CHRISTOPH, Thomas**
  **52080 Aachen (DE)**
- **KÖGEL, Babette-Yvonne**
  **52379 Langerwehe-Hamich (DE)**
- **SCHÜTTLER, Aachim**
  **52074 Aachen (DE)**
- **ZIMMER, Oswald**
  **52146 Würselen (DE)**

(74) Vertreter: **Kutzenberger, Helga**
**Kutzenberger & Wolff,**
**Theodor-Heuss-Ring 23**
**50668 Köln (DE)**

(56) Entgegenhaltungen:
**US-A- 4 426 378** **US-A- 5 686 420**
**US-A- 5 811 582**

- **DATABASE BIOSIS [Online] BIOSCIENCES INFORMATION SERVICE, PHILADELPHIA, PA, US; November 1995 (1995-11) BORISOVA E V ET AL: "Thyrotropin-releasing hormone in the mechanisms of resistance and development of dependence on morphine." Database accession no. PREV199800168994 XP001029026 & ZHURNAL VYSSHEI NERVNOI DEYATEL'NOSTI IMENI I. P. PAVLOVA, Bd. 45, Nr. 6, November 1995 (1995-11), Seiten 1174-1181, XP001029026 ISSN: 0044-4677**
- **ZHUKOV V N; YAKIMOVA K S; SHAMYAKINA I Y: "Hyperthermia and antinoceptive activity of thyrotropin-releasing hormone and morphine following central administration in rats." ACTA PHYSIOLOGICA ET PHARMACOLOGICA BULGARICA, Bd. 14, Nr. 2, 1988, Seiten 18-23, XP001029231**
- **PILLAI N P ET AL: "THE EFFECT OF TRH AND MORPHINE ON GASTROINTESTINAL TRANSIT" PEPTIDES (ELMSFORD), Bd. 5, Nr. 6, 1984, Seiten 1055-1060, XP001029069 ISSN: 0196-9781**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft eine Wirkstoffkombination enthaltend eine Verbindung mit opioider Wirksamkeit und wenigstens eine weitere Verbindung der allgemeinen Formel I und/oder eines ihrer Diastereomeren und/oder eines ihrer Enantiomeren und/oder wenigstens ein entsprechendes physiologisch verträgliches Salz, Arzneimittel enthaltend diese Wirkstoffkombination, Arzneimittelformulierungen enthaltend diese Wirkstoffkombination sowie die Verwendung dieser Wirkstoffkombination zur Herstellung eines Arzneimittels.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie z.B. das Morphin, sind bei der Therapie starker bis sehr starker Schmerzen wirksam, weisen jedoch unerwünschte Begleiterscheinungen, wie z.B. Atemdepression, Übelkeit, Erbrechen, Abhängigkeit, Sedierung, Obstipation oder Toleranzentwicklung auf.

Die der Erfindung zugrundeliegende Aufgabe bestand daher darin, analgetisch wirksame Arzneimittel zur Verfügung zu stellen, die sich zur Behandlung von starken bis sehr starken Schmerzen eignen. Darüber hinaus sollten diese Arzneimittel möglichst wenig Begleiterscheinungen der bekannten Opioid-Analgetika, wie z.B. Atemdepression, Übelkeit, Erbrechen, Abhängigkeit, Sedierung, Obstipation oder Toleranzentwicklung aufweisen.

Erfindungsgemäß wird diese Aufgabe durch die Bereitstellung einer neuen Wirkstoffkombination gelöst, die

A) eine Verbindung mit opioider Wirksamkeit und/oder wenigstens eines ihrer physiologisch verträglichen Salze und

B) wenigstens eine Verbindung der allgemeinen Formel I,

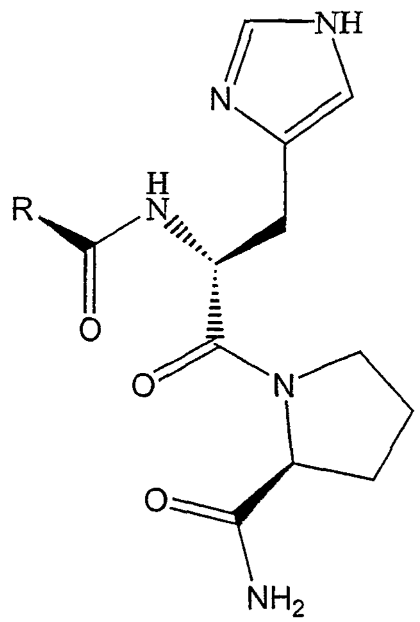

I

worin der Rest R für eine der nachfolgenden Gruppen a) bis e)

a)

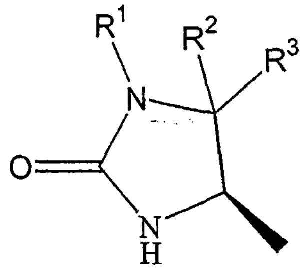

b)

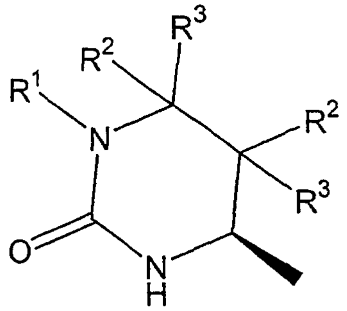

c)

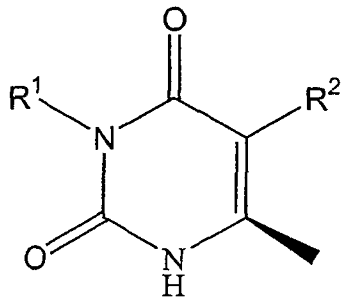

d)

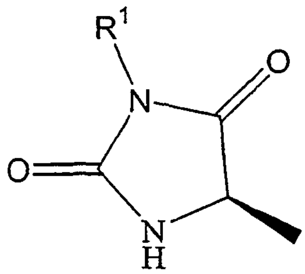

e)

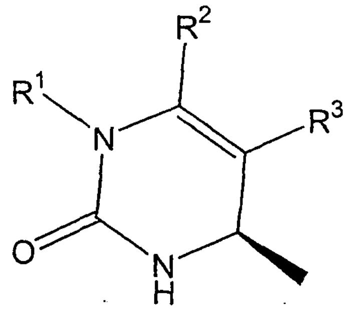

steht, in denen jeweils die Reste $R^1$, $R^2$, $R^3$, gleich oder verschieden, H oder einen $CH_3$-Rest bedeuten und/oder eines ihrer Enantiomeren und/oder eines ihrer Diastereomeren und/oder wenigstens ein entsprechendes physiologisch verträgliches Salz enthält.

Die erfindungsgemäße Wirkstoffkombination weist überraschenderweise eine ausgeprägte analgetische Wirksamkeit auf, wobei die unerwünschten Begleiterscheinungen, die durch die alleinige Verabreichung von Verbindungen mit opioider Wirksamkeit hervorgerufen werden, nicht mehr oder nur sehr schwach auftreten.

Die Herstellung der Verbindungen der allgemeinen Formel I, ihrer Enantiomeren, ihrer Diastereomeren sowie die Herstellung der entsprechenden physiologisch verträglichen Salze kann gemäß der Offenbarung in DE-PS-2449167, EP-0 429 245 oder J. Med. Chem., 1990, 33(8), Seiten 2130 ff durch Umsetzung der entsprechenden, dem Fachmann bekannten Carbonsäuren erfolgen. Die Herstellung der Verbindungen mit opioider Wirksamkeit sowie der entsprechenden Salze ist ebenfalls aus der Literatur bekannt, z.B. aus E. Friderichs, T. Christoph, H. Buschmann, "Analgesics and Antipyretics", Ullmann's Encyclopedia of Industrial Chemistry, Sith Edition on CD-ROM, Wiley-VCH, Weinheim, 2000. Die entsprechenden Offenbarungen werden hiermit als Referenz eingeführt.

Als physiologisch verträgliches Salz der Verbindungen der allgemeinen Formel I und/oder deren Enantiomeren und/oder deren Diastereomeren kann vorzugsweise das Hydrochlorid, Hydrobromid, Sulfat, Sulfonat, Phosphat, Tartrat, Embonat, Formiat, Acetat, Propionat, Benzoat, Oxalat, Succinat, Citrat, Glutamat, Fumarat, Aspartat, Glutarat, Stearat, Butyrat, Malonat, Lactat, Mesylat oder ein Gemisch aus wenigstens zwei dieser Salze eingesetzt werden.

Die erfindungsgemäße Wirkstoffkombination kann die Verbindungen der allgemeinen Formel I, deren Diastereomere, Enantiomere und deren physiologisch verträgliche Salze, sowohl einzeln als auch in Form eines Gemisches aus wenigstens zwei dieser Verbindungen enthalten. Vorzugsweise enthält die erfindungsgemäße Wirkstoffkombination eine Verbindung der allgemeinen Formel I, deren Enantiomer, deren Diastereomer oder ein entsprechendes physiologisch verträgliches Salz als Komponente B.

In einer bevorzugten Ausführungsform der vorliegenden Erfindung enthält die Wirkstoffkombination als Komponente B) eine Verbindung der allgemeinen Formel I, worin der Rest R für die Gruppe a) steht und die Reste $R^1$, $R^2$ und $R^3$ jeweils H bedeuten und/oder eines ihrer Enantiomeren und/oder eines ihrer Diastereomeren und/oder ein entsprechendes physiologisch verträgliches Salz.

In einer weiteren bevorzugten Ausführungsform der vorliegenden Erfindung enthält die erfindungsgemäße Wirkstoffkombination als Komponente B) eine Verbindung der allgemeinen Formel I, worin der Rest R für die Gruppe c) steht und $R^1 = CH_3$ und $R^2 = H$ bedeutet und/oder eines ihrer Enantiomeren und/oder eines ihrer Diastereomeren und/oder ein entsprechendes physiologisch verträgliches Salz.

Als Verbindung mit opioider Wirksamkeit können Verbindungen mit schwacher, starker oder sehr starker opioider Wirksamkeit, d.h. entsprechender analgetischer Wirksamkeit, eingesetzt werden.

Als Verbindungen mit schwacher opioider Wirksamkeit werden vorzugsweise Codein, Dextropropoxyphen, Dihydrocodein, Diphenoxylat, Ethylmorphin, Meptazinol, Nalbuphin, Pethidin (Meperidin), Tilidin, Tramadol oder Viminol eingesetzt, als Verbindungen mit starker opioider Wirksamkeit werden bevorzugt Butorphanol, Dextromoramid, Dezocin, Diacetylmorphin (Heroin), Hydrocodon, Hydromorphon, Ketobemidon, Levomethadon, Levomethadyl-Acetat, Levorphanol, Morphin, Nalorphin, Oxycodon, Pentazocin oder Piritramid und als Verbindungen mit sehr starker opioider Wirksamkeit werden vorzugsweise Alfentanil, Buprenorphin, Etorphin, Fentanyl, Remifentanil oder Sufentanil eingesetzt.

Als physiologisch verträgliches Salz der Verbindung mit opioder Wirksamkeit kann bevorzugt das Hydrochlorid, Hydrobromid, Sulfat, Sulfonat, Phosphat, Tartrat, Embonat, Formiat, Acetat, Propionat, Benzoat, Oxalat, Succinat, Citrat, Glutamat, Fumarat, Aspartat, Glutarat, Stearat, Butyrat, Malonat, Lactat, Mesylat oder ein Gemisch aus wenigstens zwei dieser Salze eingesetzt werden.

In einer besonders bevorzugten Ausführungsform der vorliegenden Erfindung enthält die neue Wirkstoffkombination als Verbindung mit opioider Wirksamkeit Morphin oder Fentanyl und/oder wenigstens ein entsprechendes physiologisch verträgliches Salz.

Ganz besonders bevorzugt enthält die erfindungsgemäße Wirkstoffkombination als Komponente A) Morphin oder Fentanyl und als Komponente B) eine Verbindung der allgemeinen Formel I, worin der Rest R für die Gruppe a) steht und die Reste $R^1$, $R^2$ und $R^3$ jeweils H bedeuten und/oder eines ihrer Enantiomeren und/oder eines ihrer Diastereomeren und/oder ein entsprechendes physiologisch verträgliches Salz.

In einer weiteren ganz besonders bevorzugten Ausführungsform enthält die erfindungsgemäße Wirkstoffkombination als Komponente A) Morphin oder Fentanyl und als Komponente B) eine Verbindung der allgemeinen Formel I, worin der Rest R für die Gruppe c) steht, der Rest $R^1$ für $CH_3$ steht und der Rest $R^2$ H bedeutet und/oder eines ihrer Enantiomeren und/oder eines ihrer Diastereomeren und/oder ein entsprechendes physiologisch verträgliches Salz.

Enthält die erfindungsgemäße Wirkstoffkombination als Komponente A) eine Verbindung mit schwacher opioider Wirksamkeit, so sollte das Gewichtsverhältnis der Komponente B) zu Komponente A) vorzugsweise im Bereich von 1 : 0,02 bis 1 : 10, besonders bevorzugt im Bereich von 1 : 0,1 bis 1 : 5 und ganz besonders bevorzugt im Bereich von 1 : 0,5 bis 1 : 2,5 liegen.

Ist die Komponente A) eine Verbindung mit starker opioider Wirksamkeit, so sollte das Gewichtsverhältnis der Komponente B) zu Komponente A) bevorzugt im Bereich von 1 : 0,002 bis 1 : 1, besonders bevorzugt im Bereich von 1 : 0,005 bis 1 : 0,5 und ganz besonders bevorzugt im Bereich von 1 : 0,01 bis 1 : 0,25 liegen.

Für Verbindungen mit sehr starker opioider Wirksamkeit als Komponente A) sollte das Gewichtsverhältnis der Komponente B) zu Komponente A) in der erfindungsgemäßen Wirkstoffkombination vorzugsweise im Bereich von 1 : 0,0002 bis 1 : 0,1 besonders bevorzugt im Bereich von 1 : 0,0005 bis 1 : 0,05, ganz besonders bevorzugt im Bereich von 1 : 0,001 bis 1 : 0,025 liegen.

Ein weiterer Gegenstand der vorliegenden Erfindung sind auch Arzneimittel enthaltend die erfindungsgemäße Wirkstoffkombination und gegebenenfalls weitere Wirkstoffe und/oder Hilfsstoffe.

Vorzugsweise werden diese erfindungsgemäßen Arzneimittel zur Bekämpfung von Schmerzen, besonders bevorzugt zur Bekämpfung von chronischen und/oder akuten Schmerzen eingesetzt.

Ein weiterer Gegenstand der vorliegenden Erfindung sind auch Arzneimittelformulierungen in unterschiedlichen Darreichungsformen, die die erfindungsgemäße Wirkstoffkombination und gegebenenfalls weitere Wirkstoffe und/oder Hilfsstoffe enthalten.

Bevorzugte Arzneimittelformulierungen sind Tabletten, Kautabletten, Kaugummis, Dragees, Kapseln, Tropfen, Säfte, Sirupe, Suppositorien, transmucale therapeutische Systeme, transdermale therapeutische Systeme, Lösungen, Emulsionen, Suspensionen, leicht rekonstituierbare Trockenzubereitungen, Pulver oder Sprays. Besonders bevorzugte Arzneimittelformulierungen sind Tabletten, Kapseln, Tropfen oder Lösungen.

In einer weiteren bevorzugten Ausführungsform liegen die erfindungsgemäßen Arzneimittelformulierungen in multipartikulärer Form, vorzugsweise als Mikrotabletten, Mikrokapseln, Mikrospheroide, Ionenaustauscherresinate, Granulate, Wirkstoffkristalle oder Pellets, besonders bevorzugt als Mikrotabletten, Granulate oder Pellets vor. Pellets im Sinne der vorliegenden Erfindung umfassen auch durch Extrusion und/oder Spheronisation hergestellte Pellets oder Aufbaupellets.

Vorzugsweise eignen sich die erfindungsgemäßen Arzneimittelformulierungen zur oralen, intravenösen, intramuskulären, subkutanen, intrathekalen, epiduralen, bukkalen, sublingualen, pulmonalen, rektalen, transdermalen, nasalen oder intracerebroventrikularen Applikation, wobei Arzneimittelformulierungen zur oralen oder intravenösen Applikation besonders bevorzugt sind.

Für die orale Applikation eignen sich vorzugsweise Zubereitungen in Form von Tabletten, Kautabletten, Kaugummis, Dragees, Kapseln, Granulaten, Tropfen, Säften, und Sirupen. Zur buccalen Applikation eignet sich bevorzugt ein transmucales therapeutisches System. Für die parenterale, topische und inhalative Applikation eignen sich vorzugsweise Lösungen, Suspensionen, Emulsionen, leicht rekonstituierbare Trockenzubereitungen, Microspheroide, Sprays, Suppositorien oder Pflaster (z.B. transdermale therapeutische Systeme). Besonders bevorzugt werden zur parenteralen Applikation Suppositorien oder Lösungen, zur topischen Applikation transdermale therapeutische Systeme und zur inhalativen Applikation Pulver oder Lösungen eingesetzt.

Zur Zubereitung der erfindungsgemäßen Arzneimittelformulierungen können neben einer erfindungsgemäßen Wirkstoffkombination bevorzugt Trägermaterialien, Füllstoffe, Lösungsmittel, Verdünnungsmittel, Farbstoffe, Aromastoffe, Bindemittel oder Gemische aus wenigstens zwei dieser Materialien eingesetzt werden. Die Auswahl dieser Hilfsstoffe sowie deren Mengen hängt davon ab, auf welche Art und Weise das Arzneimittel appliziert werden soll. Dem Fachmann sind die für die jeweilige Applikationsform geeigneten Hilfsstoffe sowie deren Mengen bekannt. Die Herstellung der erfindungsgemäßen Arzneimittelformulierungen kann nach den üblichen, dem Fachmann bekannten Methoden erfolgen.

Die erfindungsgemäßen Arzneimittelformulierungen können auch wenigstens eine der Wirkstoff-Komponenten A) oder B) in retardierter Form enthalten.

Die Retardierung der jeweiligen Wirkstoff-Komponente erfolgt vorzugsweise durch einen retardierenden Überzug, durch Fixierung an einem Ionenaustauscherharz, durch Einbettung in eine retardierende Matrix oder durch eine Kombination aus diesen Retardierungen. Geeignete, retardierende Überzüge umfassen wasserunlösliche Wachse oder Polymere, wie z.B. Acrylharze, vorzugsweise Poly(meth)acrylate, oder wasserunlösliche Cellulosen, vorzugsweise Ethylcellulose. Diese Materialien sind aus dem Stande der Technik, z.B. Bauer, Lehmann, Osterwald, Rothgang "Überzogene Arzneiformen", Wissenschaftliche Verlagsgesellschaft mbH Stuttgart, 1988, Seite 69 ff., bekannt. Sie werden hiermit als Referenz eingeführt und gelten somit als Teil der Offenbarung.

Neben den wasserunlöslichen Polymeren können gegebenenfalls zur Einstellung der Freisetzungsgeschwindigkeit des jeweiligen Wirkstoffes die retardierenden Überzüge auch nicht retardierende, vorzugsweise wasserlösliche Polymere in Mengen bis 30 Gew.%, wie Polyvinylpyrrolidon oder wasserlösliche Cellulosen, vorzugsweise Hydroxypropylmethylcellulose oder Hydroxypropylcellulose, und/oder hydrophile Porenbildner, wie Saccharose, Natriumchlorid oder Mannitol und/oder die bekannten Weichmacher enthalten.

Außerdem kann die erfindungsgemäße Wirkstoffkombination noch weitere Überzüge aufweisen. Als Überzüge können auch solche vorhanden sein, die sich pH-abhängig auflösen. So kann erreicht werden, daß die Arzneimittelformulierung den Magentrakt unaufgelöst passiert und die erfindungsgemäße Wirkstoffkombination erst im Darmtrakt zur Freisetzung gelangt. Es können auch Überzüge verwendet werden, die der Verbesserung des Geschmackes dienen.

Eine weitere übliche Verfahrensweise der Retardierung ist die Fixierung der Wirkstoffe an Ionenaustauscherharzen. Zur Retardierung sowohl der Wirkstoff-Komponente A) als auch der Wirkstoff-Komponente B) werden Kationenaustauscherharze, vorzugsweise Polystyrolsulfonate eingesetzt.

Zur Retardierung kann die erfindungsgemäße Wirkstoffkombination auch in einer retardierenden Matrix, vorzugsweise gleichmäßig verteilt, vorliegen. Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet.
Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophobe Polymere, Wachse, Fette, langkettigen Fettsäuren, Fettalkohole oder entsprechenden Ester oder Ether oder deren Gemische. Besonders bevorzugt werden als hydrophobe Materialien Mono- oder Diglyceride von $C_{12}$-$C_{30}$-Fettsäuren und/oder $C_{12}$-$C_{30}$-Fettalkohole und/oder Wachse oder deren Gemische eingesetzt.

Es ist auch möglich, Mischungen der genannten hydrophilen und hydrophoben Materialien als retardierendes Matrixmaterial einzusetzen.

In einer weiteren bevorzugten Ausführungsform enthält die erfindungsgemäße Arzneimittelformulierung wenigstens eine der Wirkstoff-Komponenten A) oder B) neben ihrer retardierten Form auch in ihrer unretardierten Form. Durch Kombination mit dem sofort freigesetzten Wirkstoff läßt sich eine hohe Initialdosis zur schnellen Schmerzlinderung erzielen. Die langsame Freisetzung aus der retardierten Form verhindert dann ein Abklingen der analgetischen Wirkung.

Die an den Patienten zu verabreichende Menge der erfindungsgemäßen Wirkstoffkombination variiert z.B. in Abhängigkeit vom Gewicht des Patienten, von der Art der Applikation, der Indikation sowie dem Schweregrad der Erkrankung. Vorzugsweise werden die zu verabreichende Menge sowie die Freisetzung der erfindungsgemäßen Wirkstoffkombination so eingestellt, daß eine Applikation höchstens zweimal, vorzugsweise nur einmal täglich erfolgen muß.

Bei einer einmaligen Applikation pro Tag enthalten die erfindungsgemäßen Wirkstoffkombinationen vorzugsweise 0,1 bis 2000 mg, besonders bevorzugt 0,5 mg bis 1000 mg der Wirkstoff-Komponente A) und vorzugsweise 0,1 bis 100 mg, besonders bevorzugt 0,5 bis 50 mg der Wirkstoff-Komponente B).

Bei einer zweimaligen Applikation pro Tag enthalten die erfindungsgemäßen Wirkstoffkombinationen vorzugsweise 0,05 bis 1000 mg, besonders bevorzugt 0,25 bis 500 mg der Wirkstoff-Komponente A) und vorzugsweise 0,05 bis 50 mg, besonders bevorzugt 0,25 bis 25 mg der Wirkstoff-Komponente B).

Ein weiterer Gegenstand der Erfindung ist auch die Verwendung einer erfindungsgemäßen Wirkstoffkombination zur Herstellung eines Arzneimittels, vorzugsweise zur Herstellung eines Arzneimittels zur Bekämpfung von Schmerzen, besonders bevorzugt zur Bekämpfung von chronischen und/oder akuten Schmerzen, da die erfindungsgemäßen Wirkstoffkombinationen eine sehr gute Wirkung bei der Bekämpfung von starken bis sehr starken Schmerzen, insbesondere bei der Bekämpfung von chronischen und/oder akuten Schmerzen zeigen. Die erfindungsgemäße Wirkstoffkombination zeigt außerdem überraschenderweise eine gegenüber der Verabreichung von ausschließlich opioiden Wirkstoffen verbesserte analgetische Wirksamkeit und weist geringere unerwünschte Begleiterscheinungen auf.

Vorteilhafterweise kann daher die für eine effektive Schmerzbekämpfung erforderliche Dosis der Verbindung mit opioider Wirksamkeit gesenkt werden. Dies führt dazu, daß die mit der Applikation einer solchen Verbindung mit opioider Wirksamkeit üblicherweise einhergehenden unerwünschten Begleiterscheinungen, wie z.B. Atemdepression, Erbrechen, Abhängigkeit, Sedierung, Obstipation oder Toleranzentwicklung nicht mehr oder nur sehr schwach auftreten.

**Pharmakologische Untersuchungen:**

Brennstrahl-Test an der Ratte:

Die analgetische Wirksamkeit der erfindungsgemäßen Wirkstoffkombinationen sowie der Vergleichslösungen wurde im Brennstrahl-(Tail-flick)-Test an der Ratte nach D'Amour and Smith, J. Pharm. Exp. Ther. Band 72, Seiten 74-79, 1941 untersucht. Hierzu wurden weibliche Sprague Dawley Ratten (Janvier, Frankreich) mit einem Gewicht zwischen 120 und 150 g verwendet.
Die Ratten wurden jeweils einzeln in einen Testkäfig gesetzt und die Schwanzbasis dem fokussierten Wärmestrahl einer elektrischen Lampe (Tail-flick-Typ 50/08/1.bc, Labtec, Dr. Hess) ausgesetzt. Die Lampenintensität wurde so eingestellt, daß die Zeit vom Einschalten der Lampe bis zum plötzlichen Wegzucken des Schwanzes (Schmerzlatenz) bei unbehandelten Ratten 3 bis 5 Sekunden betrug. Vor der Applikation der erfindungsgemäßen Wirkstoffkombinationen sowie der Vergleichslösungen wurden die Ratten innerhalb von fünf Minuten zweimal vorgetestet und der Mittelwert dieser Messungen als Vortestmittelwert berechnet. Die Lösungen der erfindungsgemäßen Wirkstoffkombinationen und die Vergleichslösungen wurden dann intravenös appliziert. Die Schmerzmessung wurde jeweils 10, 20, 40 und 60 Minuten nach der intravenösen Applikation durchgeführt. Die analgetische Wirkung wurde als Zunahme der Schmerzlatenz (% des maximal möglichen antinociceptiven Effektes) nach der folgenden Formel bestimmt:

$$[(T_1-T_0)/(T_2-T_0)] \times 100$$

Hierbei ist die Zeit $T_0$ die Latenzzeit vor der Applikation, die Zeit $T_1$ die Latenzzeit nach der Applikation der Wirkstoffkombination und die Zeit $T_2$ die maximale Expositionsdauer (12 Sekunden).
Die folgenden Beispiele dienen der Erläuterung der Erfindung, schränken aber den allgemeinen Erfindungsgedanken nicht ein.

**Beispiele:**

**Beispiel 1:**

Zur Untersuchung der analgetischen Wirkung der Wirkstoffkombination enthaltend als Komponente A) Morphin und als Komponente B) die Verbindung der allgemeinen Formel I, in der der Rest R für die Gruppe a) steht und die Reste $R^1$, $R^2$ und $R^3$ jeweils H bedeuten, wurde einer ersten Gruppe von 10 Ratten jeweils eine 0,9%ige Kochsalzlösung enthaltend 1,46 mg Morphin pro kg Körpergewicht der Ratte und 21,5 mg der genannten Komponente B pro kg Körpergewicht der Ratte intravenös verabreicht.

**Vergleichsbeispiel 1:**

Zum Vergleich wurde einer zweiten Gruppe von 10 Ratten jeweils eine 0,9%ige -Kochsalzlösung enthaltend nur 1,46 mg Morphin pro kg Körpergewicht der Ratte intravenös verabreicht.
Die Ergebnisse dieser Untersuchungen sind in der **Figur 1** dargestellt.

**Beispiel 2:**

Zur Untersuchung der analgetischen Wirkung einer erfindungsgemäßen Wirkstoffkombination enthaltend als Komponente A) Morphin und als Komponente B) die Verbindung der allgemeinen Formel I, in der der Rest R für die Gruppe c) steht, der Rest $R^1$ für $CH_3$ steht und der Rest $R^2$ H bedeutet, wurde einer dritten Gruppe von 10 Ratten jeweils eine 0,9%ige Lösung enthaltend 1,46 mg Morphin pro kg Körpergewicht der Ratte und 46,4 mg von der gennanten Komponente B pro kg Körpergewicht der Ratte intravenös verabreicht.
Die Ergebnisse dieser Untersuchung sind zusammen mit dem Ergebnis der Vergleichslösung gemäß Vergleichsbeispiel 1 in **Figur 2** dargestellt. Wie aus den Figuren 1 und 2 ersichtlich ist, zeigt die Vergleichslösung gemäß dem Vergleichsbeispiel 1, die allein Morphin enthält, bereits eine gute analgetische Wirksamkeit.
Wie ebenfalls aus den Figuren 1 und 2 ersichtlich ist, bewirkt die Applikation der Lösungen gemäß den Beispielen 1 und 2 enthaltend die erfindungsgemäßen Wirkstoffkombinationen aus Morphin und der jeweiligen gennanten Wirkstoff-Komponente B) eine gegenüber der alleinigen Applikation von Morphin jeweils stark verbesserte analgetische Wirksamkeit.

**Patentansprüche**

**1.** Wirkstoffkombination enthaltend

A) eine Verbindung mit opioider Wirksamkeit und/oder wenigstens eines ihrer physiologisch verträglichen Salze und

B) wenigstens eine Verbindung der allgemeinen Formel I,

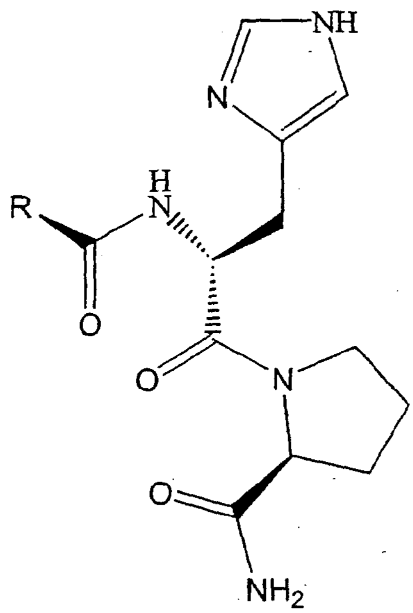

I

worin der Rest R für eine der nachfolgenden Gruppen a) bis e)

a)

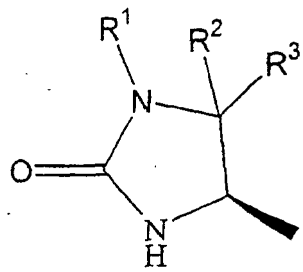

b)

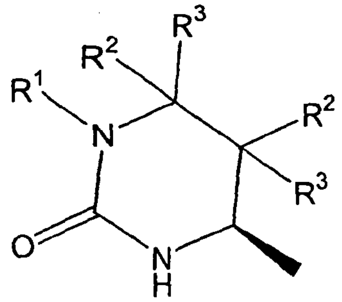

c)

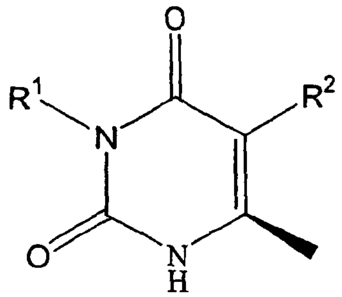

d)

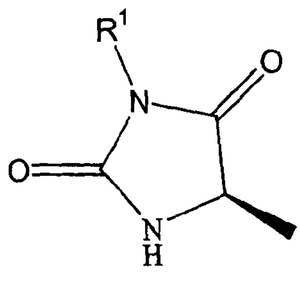

e)

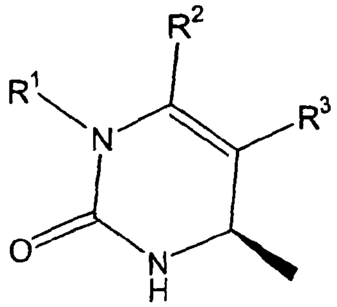

steht und jeweils die Reste $R^1$, $R^2$, $R^3$, jeweils gleich oder verschieden, einen H- oder $CH_3$-Rest bedeuten und/oder eines ihrer Enantiomeren und/oder eines ihrer Diastereomeren und/oder wenigstens ein entsprechendes physiologisch verträgliches Salz enthält.

2. Wirkstoffkombination gemäß Anspruch 1, **dadurch gekennzeichnet, daß** der Rest R für die Gruppe a) steht und die Reste $R^1$, $R^2$ und $R^3$ jeweils H bedeuten.

3. Wirkstoffkombination gemäß Anspruch 1, **dadurch gekennzeichnet, daß** der Rest R für die Gruppe c) steht, $R^1$ für $CH_3$ steht und der Rest $R^2$ H bedeutet.

4. Wirkstoffkombination gemäß einem der Ansprüche 1 bis 3, **dadurch gekennzeichnet, daß** als Komponente A) eine Verbindung mit schwacher, starker oder sehr starker opioider Wirksamkeit vorliegt.

5. Wirkstoffkombination gemäß Anspruch 4, **dadurch gekennzeichnet, daß** als Verbindung mit schwacher opioider Wirksamkeit Codein, Dextropropoxyphen, Dihydrocodein, Diphenoxylat, Ethylmorphin, Meptazinol, Nalbuphin, Pethidin (Meperidin), Tilidin, Tramadol oder Viminol vorliegt.

6. Wirkstoffkombination gemäß Anspruch 5, **dadurch gekennzeichnet, daß** das Gewichtsverhältnis von Komponente B) zu Komponente A) im Bereich von 1 : 0,02 bis 1 : 10, vorzugsweise im Bereich von 1 : 0,1 bis 1 : 5, besonders bevorzugt im Bereich von 1 : 0,5 bis 1 : 2,5 liegt.

7. Wirkstoffkombination gemäß Anspruch 4, **dadurch gekennzeichnet, daß** als Verbindung mit stark opioider Wirksamkeit Butorphanol, Dextromoramid, Dezocin, Diacetylmorphin (Heroin), Hydrocodon, Hydromorphon, Ketobemidon, Levomethadon, Levomethadyl-Acetat, Levorphanol, Morphin, Nalorphin, Oxycodon, Pentazocin oder Piritramid, vorzugsweise Morphin vorliegt.

8. Wirkstoffkombination gemäß Anspruch 7, **dadurch gekennzeichnet, daß** das Gewichtsverhältnis von Komponente B) zu Komponente A) im Bereich von 1: 0,002 bis 1 : 1, vorzugsweise im Bereich von 1 : 0,005 bis 1 : 0,5 besonders bevorzugt im Bereich von 1 : 0,01 bis 1 : 0,25 liegt.

9. Wirkstoffkombination gemäß Anspruch 4, **dadurch gekennzeichnet, daß** als Verbindung mit sehr starker opioider Wirksamkeit Alfentanil, Buprenorphin, Etorphin, Fentanyl, Remifentanil oder Sufentanil, vorzugsweise Fentanyl vorliegt.

10. Wirkstoffkombination gemäß Anspruch 9, **dadurch gekennzeichnet, daß** das Gewichtsverhältnis von Komponente B) zu Komponente A) im Bereich von 1 : 0,0002 bis 1 : 0,1, vorzugsweise im Bereich von 1 : 0,0005 bis 1 : 0,05, besonders bevorzugt im Bereich von 1 : 0,001 bis 1 : 0,025 liegt.

11. Wirkstoffkombination gemäß einem der Ansprüche 1 bis 10, **dadurch gekennzeichnet, daß** als physiologisch verträgliches Salz der Verbindung mit opioider Wirksamkeit ein Hydrochlorid, Hydrobromid, Sulfat, Sulfonat, Phosphat, Tartrat, Embonat, Formiat, Acetat, Propionat, Benzoat, Oxalat, Succinat, Citrat, Glutamat, Fumarat, Aspartat,

Glutarat, Stearat, Butyrat, Malonat, Lactat, Mesylat oder ein Gemisch aus wenigstens zwei dieser Salze vorliegt.

**12.** Wirkstoffkombination gemäß einem der Ansprüche 1 bis 11, **dadurch gekennzeichnet, daß** als physiologisch verträgliches Salz der Verbindung der allgemeinen Formel I und/oder deren Enantiomeren und/oder deren Diastereomeren ein Hydrochlorid, Hydrobromid, Sulfat, Sulfonat, Phosphat, Tartrat, Embonat, Formiat, Acetat, Propionat, Benzoat, Oxalat, Succinat, Citrat, Glutamat, Fumarat, Aspartat, Glutarat, Stearat, Butyrat, Malonat, Lactat, Mesylat oder ein Gemisch aus wenigstens zwei dieser Salze vorliegt.

**13.** Arzneimittel enthaltend eine Wirkstoffkombination gemäß einem der Ansprüche 1 bis 12 und gegebenenfalls weitere Wirkstoffe und/oder Hilfsstoffe.

**14.** Arzneimittelformulierung enthaltend eine Wirkstoffkombination gemäß einem der Ansprüche 1 bis 12 und ggf. weitere Wirkstoffe und/oder Hilfsstoffe.

**15.** Arzneimittelformulierung gemäß Anspruch 14, **dadurch gekennzeichnet, daß** sie in Form von Tabletten, Kautabletten, Kaugummis, Dragees, Kapseln, Suppositorien, trandermalen therapeutischen Systemen, transmucosalen therapeutischen Systemen, Tropfen oder als Saft, Sirup, Lösung, Emulsion, Suspension, leicht rekonstituierbare Trockenzubereitung, Pulver oder als Spray, vorzugsweise in Form von Tabletten, Kapseln, Tropfen oder als Lösung, vorliegt.

**16.** Arzneimittelformulierung gemäß Anspruch 14, **dadurch gekennzeichnet, daß** sie in multipartikulärer Form, vorzugsweise in Form von Mikrotabletten, Mikrokapseln, Mikrospheroiden, Ionenaustauscherresinaten, Granulaten, Wirkstoffkristallen oder Pellets, besonders bevorzugt in Form von Mikrotabletten, Granulaten oder Pellets vorliegt.

**17.** Arzneimittelformulierung gemäß einem der Ansprüche 14 bis 16 zur oralen, intravenösen, intramuskulären, subkutanen, intrathekalen, epiduralen, bukkalen, sublingualen, rektalen, pulmonalen, transdermalen, nasalen oder intracerebroventrikularen, bevorzugt zur oralen oder intravenösen Applikation.

**18.** Arzneimittelformulierung gemäß einem der Ansprüche 14 bis 17, **dadurch gekennzeichnet, daß** wenigstens eine der Wirkstoff-Komponenten A) oder B) in retardierter Form vorliegt.

**19.** Arzneimittelformulierung gemäß Anspruch 18, **dadurch gekennzeichnet, daß** die Retardierung durch einen retardierenden Überzug, Fixierung an einem Ionenaustauscherharz, Einbettung in eine retardierende Matrix oder einer Kombination daraus erfolgt ist.

**20.** Arzneimittelformulierung gemäß Anspruch 19, **dadurch gekennzeichnet, daß** der Überzug auf einem wasserunlöslichen Polymeren oder Wachs basiert.

**21.** Arzneimittelformulierung gemäß Anspruch 20, **dadurch gekennzeichnet, daß** als wasserunlösliches Polymeres ein (Poly)acrylharz oder Cellulosederivat, vorzugsweise Alkylcellulose, eingesetzt ist.

**22.** Arzneimittelformulierung gemäß Anspruch 21, **dadurch gekennzeichnet, daß** Ethylcellulose und/oder ein Poly (meth)acrylat als Polymeres eingesetzt ist.

**23.** Arzneimittelformulierung gemäß Anspruch 19, **dadurch gekennzeichnet, daß** die Matrix hydrophiles Matrixmaterial, vorzugsweise Polymere, besonders bevorzugt Celluloseether, Celluloseester und/oder Acrylharze, ganz besonders bevorzugt Ethylcellulose, Hydroxypropylmethylcellulose, Hydroxypropylcellulose, Hydroxymethylcellulose, Poly(meth)acrylsäure und/oder deren Salze, Amide und/oder Ester enthält.

**24.** Arzneimittelformulierung gemäß Anspruch 19 oder 23, **dadurch gekennzeichnet, daß** die Matrix hydrophobes Matrixmaterial, vorzugsweise Polymere, Wachse, Fette, langkettigen Fettsäuren, Fettalkohole oder entsprechende Ester oder Ether oder deren Gemische, besonders bevorzugt Mono- oder Diglyceride von $C_{12}$-$C_{30}$-Fettsäuren und/oder $C_{12}$-$C_{30}$-Fettalkohole und/oder Wachse oder deren Gemische enthält.

**25.** Arzneimittelformulierung gemäß Anspruch 19, **dadurch gekennzeichnet, daß** als Kationenaustauscherharze Polystryrolsulfonate eingesetzt sind.

**26.** Arzneimittelformulierung nach einem oder mehreren der Ansprüche 18 bis 25, **dadurch gekennzeichnet, daß**

wenigstens eine der Wirkstoff-Komponenten A) oder B) neben der retardierten Form auch in der unretardierten Form vorliegt.

**27.** Verwendung einer Wirkstoffkombination gemäß einem der Ansprüche 1 bis 12 zur Herstellung eines Arzneimittels zur Bekämpfung von Schmerzen.

**28.** Verwendung gemäß Anspruch 27 zur Herstellung eines Arzneimittels zur Bekämpfung von chronischen Schmerzen.

**29.** Verwendung gemäß Anspruch 27 zur Herstellung eines Arzneimittels zur Bekämpfung von akuten Schmerzen.

## Claims

**1.** Active substance combination containing

A) a compound with an opioid effect and/or at least one of the physiologically acceptable salts thereof and
B) at least one compound of the general formula I

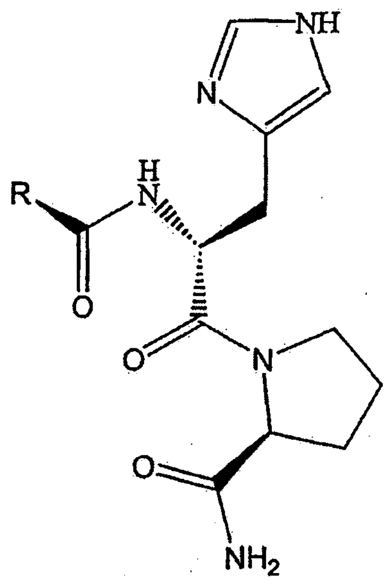

I

with the residue R standing for one of the following groups a) to e)

a)

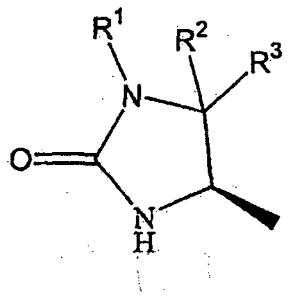

b)

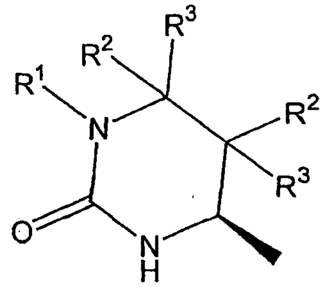

c)

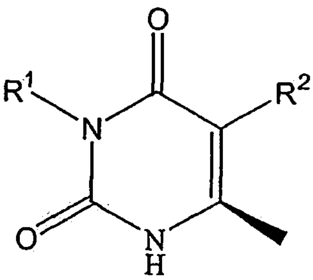

d)

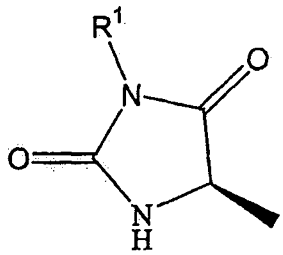

e)

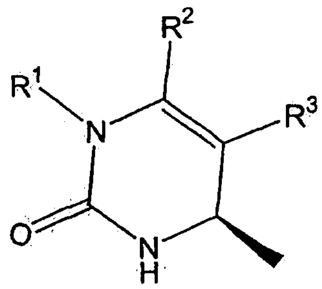

and the residues $R^1$, $R^2$, $R^3$ each, identical or different, meaning an H or $CH_3$ residue, and/or one of the enantiomers thereof and/or one of the diastereomers thereof and/or at least one corresponding physiologically acceptable salt.

2. Active substance combination according to claim 1, **characterised in that** the residue R stands for the group a) and the residues $R^1$, $R^2$ and $R^3$ each mean H.

3. Active substance combination according to claim 1, **characterised in that** the residue R stands for the group c), $R^1$ stands for $CH_3$ and the residue $R^2$ means H.

4. Active substance combination according one of claims 1 to 3, **characterised in that**, as component A), a compound with a weak, strong or very strong opioid effect is present.

5. Active substance combination according to claim 4, **characterised in that**, as a compound with a weak opioid effect, codeine, dextropropoxyphene, dihydrocodeine, diphenoxylate, ethylmorphine, meptazinol, nalbuphine, pethidine (meperidine), tilidine, tramadol or viminol is present.

6. Active substance combination according to claim 5, **characterised in that** the weight ratio of component B) to component A) is in the range from 1:0.02 to 1:10, preferably in the range from 1:0.1 to 1:5, particularly preferably in the range from 1:0.5 to 1:2.5.

7. Active substance combination according to claim 4, **characterised in that**, as a compound with a strong opioid effect, butorphanol, dextromoramide, dezocine, diacetylmorphine (heroin), hydrocodone, hydromorphone, ketobemidone, levomethadone, levomethadyl acetate, levorphanol, morphine, nalorphine, oxycodone, pentazocine or piritramide, preferably morphine, is present.

8. Active substance combination according to claim 7, **characterised in that** the weight ratio of component B) to component A) is in the range from 1:0.002 to 1:1, preferably in the range from 1:0.005 to 1:0.5, particularly preferably in the range from 1:0.01 to 1:0.25.

9. Active substance combination according to claim 4, **characterised in that**, as a compound with a very strong opioid effect, alfentanil, buprenorphine, etorphine, fentanyl, remifentanil or sufentanil, preferably fentanyl, is present.

10. Active substance combination according to claim 9, **characterised in that** the weight ratio of component B) to component A) is in the range from 1:0.0002 to 1:0.1, preferably in the range from 1:0.0005 to 1:0.05, particularly preferably in the range from 1:0.001 to 1:0.025.

11. Active substance combination according to one of claims 1 to 10, **characterised in that**, as a physiologically acceptable salt of the compound with an opioid effect, a hydrochloride, hydrobromide, sulfate, sulfonate, phosphate, tartrate, embonate, formate, acetate, propionate, benzoate, oxalate, succinate, citrate, glutamate, fumarate, aspartate, glutarate, stearate, butyrate, malonate, lactate, mesylate or a mixture of at least two of these salts is present.

12. Active substance combination according to one of claims 1 to 11, **characterised in that**, as a physiologically acceptable salt of the compound of the general formula I and/or the enantiomers thereof and/or the diastereomers thereof, a hydrochloride, hydrobromide, sulfate, sulfonate, phosphate, tartrate, embonate, formate, acetate, propionate, benzoate, oxalate, succinate, citrate, glutamate, fumarate, aspartate, glutarate, stearate, butyrate, malonate, lactate, mesylate or a mixture of at least two of these salts is present.

13. Medicament containing an active substance combination according to one of claims 1 to 12 and optionally further active substances and/or auxiliary substances.

14. Pharmaceutical formulation containing an active substance combination according to one of claims 1 to 12 and optionally further active substances and/or auxiliary substances.

15. Pharmaceutical formulation according to claim 14, **characterised in that** it is in the form of tablets, chewable tablets, chewing gums, sugar-coated tablets, capsules, suppositories, transdermal therapeutic systems, transmucosal therapeutic systems, drops or as succus, syrup, solution, emulsion, suspension, easily reconstitutable dry preparations, powder or spray, preferably in the form of tablets, capsules, drops or solution.

16. Pharmaceutical formulation according to claim 14, **characterised in that** it is present in multiparticulate form, preferably microtablets, microcapsules, microspheriods, ion-exchange resinates, granules, active substance crystals or pellets, particularly preferably as microtablets, granules or pellets.

17. Pharmaceutical formulation according to one of claims 14 to 16, for oral, intravenous, intramuscular, subcutaneous, intrathecal, epidural, buccal, sublingual, rectal, pulmonary, transdermal, nasal or intracerebroventricular, preferably oral or intravenous, administration.

18. Pharmaceutical formulation according to one of claims 14 to 17, **characterised in that** at least one of the active substance components A) or B) is present in delayed-release form.

19. Pharmaceutical formulation according to claim 18, **characterised in that** delayed release is achieved by a delayed-release coating, by attachment to an ion-exchange resin, by embedding in a delayed-release matrix or by a combination thereof.

20. Pharmaceutical formulation according to claim 19, **characterised in that** the coating is based on a water-insoluble polymer or wax.

21. Pharmaceutical formulation according to claim 20, **characterised in that** a poly(acrylic) resin or cellulose derivative, preferably alkyl cellulose, is used as a water-insoluble polymer.

22. Pharmaceutical formulation according to claim 21, **characterised in that** ethyl cellulose and/or a poly(meth)acrylate is used as a polymer.

23. Pharmaceutical formulation according to claim 19, **characterised in that** the matrix contains hydrophilic matrix material, preferably polymers, particularly preferably cellulose ethers, cellulose esters and/or acrylic resins, very particularly preferably ethyl cellulose, hydroxypropylmethylcellulose, hydroxypropylcellulose, hydroxymethylcellulose, poly(meth)-acrylic acid and/or the salts, amides and/or esters thereof.

24. Pharmaceutical formulation according to claim 19 or 23, **characterised in that** the matrix contains hydrophobic matrix material, preferably polymers, waxes, fats, long-chain fatty acids, fatty alcohols or corresponding esters or ethers or mixtures thereof, particularly preferably mono- or diglycerides of $C_{12}$-$C_{30}$ fatty acids and/or $C_{12}$-$C_{30}$ fatty alcohols and/or waxes or mixtures thereof.

25. Pharmaceutical formulation according to claim 19, **characterised in that** polystyrene sulfonates are used as cation-exchange resins.

26. Pharmaceutical formulation according to one or more of claims 18 to 25, **characterised in that** in addition to the delayed-release form, at least one of the active substance components A) or B) is present in the non-delayed-release form.

27. Use of an active substance combination according to one of the claims 1 to 12 for the manufacture of a medicament for the alleviation of pain.

28. Use according to claim 27 for the manufacture of a medicament for the alleviation of chronic pain.

29. Use according to claim 27 for the manufacture of a medicament for the alleviation of acute pain.

## Revendications

1. Combinaison de principes actifs, contenant

A) un composé ayant une activité opioïde, et/ou au moins l'un de ses sels compatibles d'un point de vue physiologique, et
B) au moins un composé de formule générale I

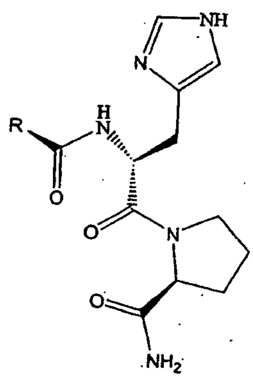

I

dans laquelle le radical R représente l'un des groupes a) à e) suivant :

a)

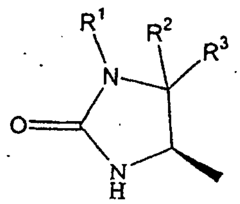

1

b)

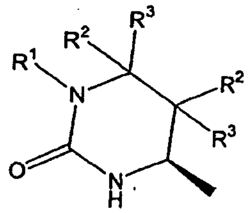

c)

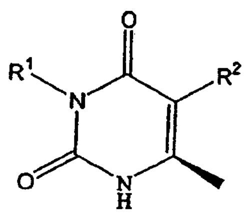

d) 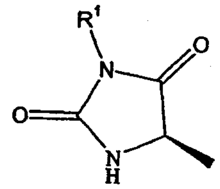

e) 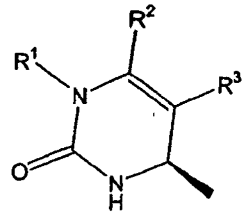

et les radicaux $R^1$, $R^2$ et $R^3$, qui sont à chaque fois identiques ou différents, représentent chacun un atome d'hydrogène ou un radical $CH_3$, et/ou un de ses énantiomères et/ou un de ses diastéréoisomères et/ou au moins un sel correspondant compatible toléré d'un point de vue physiologique.

2. Combinaison de principes actifs selon la revendication 1, **caractérisée en ce que** le radical R représente le groupe a), et chacun des radicaux $R^1$, $R^2$ et $R^3$ est un atome d'hydrogène.

3. Combinaison de principes actifs selon la revendication 1, **caractérisée en ce que** le radical R représente le groupe c), $R^1$ est $CH_3$, et le radical $R^2$ est un atome d'hydrogène.

4. Combinaison de principes actifs selon l'une des revendications 1 à 3, **caractérisée en ce que** le composant A) est un composé ayant une activité opioïde faible, forte ou très forte.

5. Combinaison de principes actifs selon la revendication 4, **caractérisée en ce que** le composé ayant une activité opioïde faible est la codéine, le dextropropoxyphène, la dihydrocodéine, le diphénoxylate, l'éthylmorphine, le meptazinol, la nalbuphine, la péthidine (mépéridine), la tilidine, la tramadol ou le viminol.

6. Combinaison de principes actifs selon la revendication 5, **caractérisée en ce que** le rapport en poids du composant B) au composant A) est compris dans la plage de 1:0,02 à 1:10, de préférence dans la plage de 1:0,1 à 1:5, plus préférablement dans la plage de 1:0,5 à 1:2,5.

7. Combinaison de principes actifs selon la revendication 4, **caractérisée en ce que** le composé ayant une forte activité opioïde est le butorphanol, le dextromoramide, la dézocine, la diacétylmorphine (héroïne), l'hydrocodone, l'hydromorphone, la cétobémidone, la lévométhadone, l'acétate de lévométhadyl, le lévorphanol, la morphine, la nalorphine, l'oxycodone, la pentazocine ou le piritramide, de préférence la morphine.

8. Combinaison de principes actifs selon la revendication 7, **caractérisée en ce que** le rapport en poids du composant B) au composant A) est compris dans la plage de 1:0,002 à 1:1, de préférence dans la plage de 1:0,005 à 1:0,5, plus préférablement dans la plage de 1:0,01 à 1:0,25.

9. Combinaison de principes actifs selon la revendication 4, **caractérisée en ce que** le composé ayant une très forte activité opioïde est l'alfentanil, la buprénorphine, l'étorphine, le fentanyl, le rémifentanil ou le sufentanil, de préférence le fentanyl.

10. Combinaison de principes actifs selon la revendication 9, **caractérisée en ce que** le rapport en poids du composant B) au composant A) est compris dans la plage de 1:0,0002 à 1:0,1, de préférence dans la plage de 1:0,0005 à 1: 0,05, plus préférablement dans la plage de 1:0,001 à 1:0,025.

**11.** Combinaison de principes actifs selon l'une des revendications 1 à 10, **caractérisée en ce que** le sel compatible d'un point de vue physiologique du composé ayant une activité opioïde est un chlorhydrate, un bromhydrate, un sulfate, un sulfonate, un phosphate, un tartrate, un embonate, un formiate, un acétate, un propionate, un benzoate, un oxalate, un succinate, un citrate, un glutamate, un fumarate, un aspartate, un glutarate, un stéarate, un butyrate, un malonate, un lactate, un mésylate, ou un mélange d'au moins deux de ces sels.

**12.** Combinaison de principes actifs selon l'une des revendications 1 à 11, **caractérisée en ce que** le sel compatible d'un point de vue physiologique du composé de formule générale I et/ou de ses énantiomères et/ou de ses diastéréoisomères est un chlorhydrate, un bromhydrate, un sulfate, un sulfonate, un phosphate, un tartrate, un embonate, un formiate, un acétate, un propionate, un benzoate, un oxalate, un succinate, un citrate, un glutamate, un fumarate, un aspartate, un glutarate, un stéarate, un butyrate, un malonate, un lactate, un mésylate, ou un mélange d'au moins deux de ces sels.

**13.** Médicament contenant une combinaison de principes actifs selon l'une des revendications 1 à 12, et éventuellement d'autres principes actifs et/ou adjuvants.

**14.** Formulation médicamenteuse contenant une combinaison de principes actifs selon l'une des revendications 1 à 12, et éventuellement d'autres principes actifs et/ou adjuvants.

**15.** Formulation médicamenteuse selon la revendication 14, **caractérisée en ce qu'**elle se présente sous forme de comprimés, de comprimés à mâcher, de gommes à mâcher, de dragées, de gélules, de suppositoires, de systèmes thérapeutiques transdermiques, de systèmes thérapeutiques transmuqueux, de gouttes, ou sous forme d'un jus, d'un sirop, d'une solution, d'une émulsion, d'une suspension, d'une préparation sèche facilement reconstituable, d'une poudre, ou sous forme d'un spray, de préférence sous forme de comprimés, de gélules, de gouttes ou sous forme d'une solution.

**16.** Formulation médicamenteuse selon la revendication 14, **caractérisée en ce qu'**elle se présente sous forme multiparticulaire, de préférence sous forme de microcomprimés, de microgélules, de microsphéroïdes, de résinates échangeurs d'ions, de granulés, de cristaux de principes actifs ou de pastilles, plus préférablement sous forme de microcomprimés, de granulés ou de pastilles.

**17.** Formulation médicamenteuse selon l'une des revendications 14 à 16 pour administration orale, intraveineuse, intramusculaire, sous-cutanée, intrathécale, épidurale, buccale, sublinguale, rectale, pulmpnaire, transdermique, nasale ou intracérébro-ventriculaire, de préférence orale ou intraveineuse.

**18.** Formulation médicamenteuse selon l'une des revendications 14 à 17, **caractérisée en ce qu'**au moins l'un des composants principes actifs A) ou B) se présente sous forme retard.

**19.** Formulation médicamenteuse selon la revendication 18, **caractérisée en ce que** l'effet retard est réalisé par un enrobage à effet retard, une fixation à une résine échangeuse d'ions, une incorporation dans une matrice à effet retard, ou une combinaison de ces techniques.

**20.** Formulation médicamenteuse selon la revendication 19, **caractérisée en ce que** l'enrobage est à base d'un polymère ou d'une cire insoluble dans l'eau.

**21.** Formulation médicamenteuse selon la revendication 20, **caractérisée en ce qu'**on utilise en tant que polymère insoluble dans l'eau une résine (poly)acrylique ou un dérivé de cellulose, de préférence une alkylcellulose.

**22.** Formulation médicamenteuse selon la revendication 21, **caractérisée en ce qu'**on utilise en tant que polymère de l'éthylcellulose et/ou un poly(méth)acrylate.

**23.** Formulation médicamenteuse selon la revendication 19, **caractérisée en ce que** la matrice contient un matériau de matrice hydrophile, de préférence un polymère, plus préférablement un éther de cellulose, un ester de cellulose et/ou des résines acryliques, encore plus préférablement de l'éthylcellulose, de l'hydroxypropylméthylcellulose, de l'hydroxypropylcellulose, de l'hydroxyméthylcellulose, du poly(acide(méth)acrylique) et/ou leurs sels, amides et/ou esters.

**24.** Formulation médicamenteuse selon la revendication 19 ou 23, **caractérisée en ce que** la matrice contient un

matériau de matrice hydrophobe, de préférence un polymère, des cires, des graisses, des acides gras à longue chaîne, des alcools gras ou des esters ou éthers correspondants, ou leurs mélanges, plus préférablement des mono- ou diglycérides d'acides gras en $C_{12}$-$C_{30}$ et/ou des alcools gras en $C_{12}$-$C_{30}$ et/ou des cires, ou leurs mélanges.

**25.** Formulation médicamenteuse selon la revendication 19, **caractérisée en ce qu'**on utilise en tant que résines échangeuses de cations des polystyrènesulfonates.

**26.** Formulation médicamenteuse selon l'une ou plusieurs des revendications 18 à 25, **caractérisée en ce qu'**au moins l'un des composants principes actifs A) ou B) se présente, outre sous forme retard, sous forme non retard.

**27.** Utilisation d'une composition de principes actifs selon l'une des revendications 1 à 12 pour préparer un médicament destiné à lutter contre la douleur.

**28.** Utilisation selon la revendication 27, pour préparer un médicament destiné à lutter contre les douleurs chroniques.

**29.** Utilisation selon la revendication 27, pour préparer un médicament destiné à lutter contre les douleurs aiguës.

Figur 1

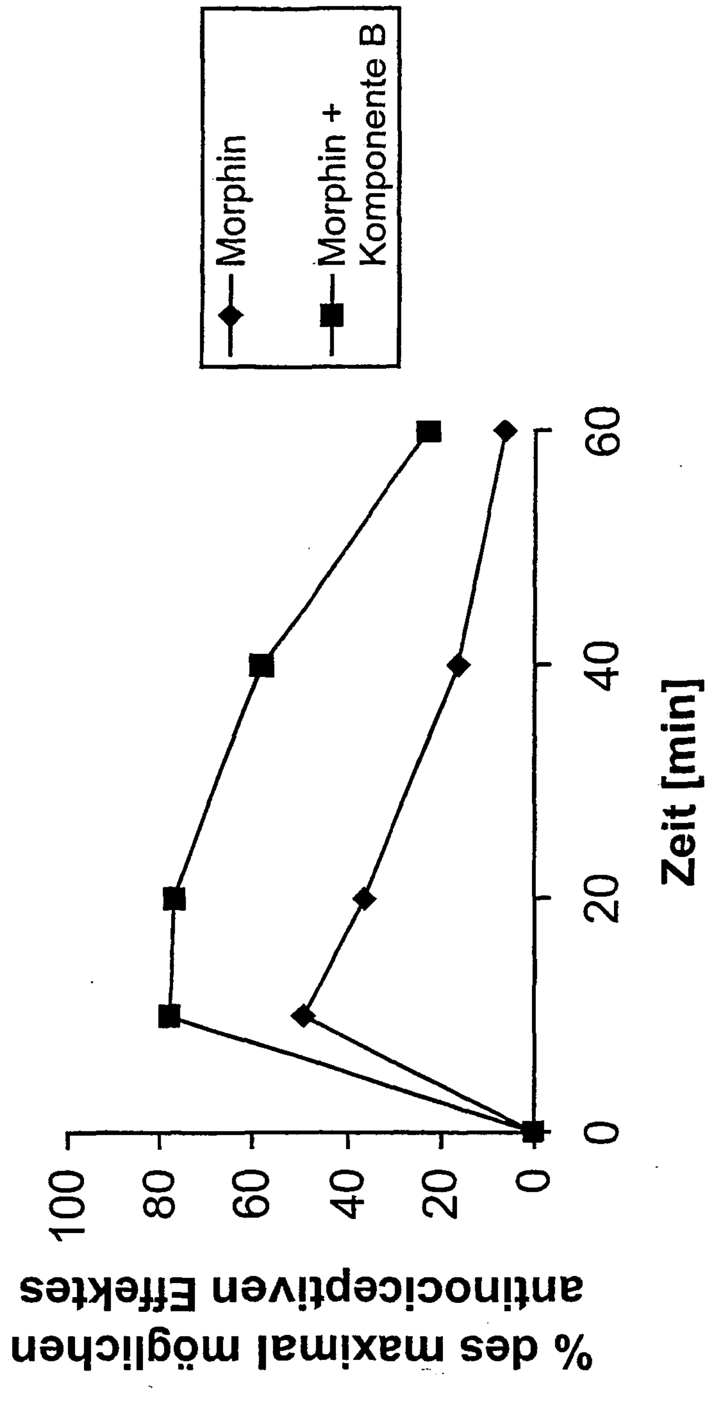
% des maximal möglichen antinociceptiven Effektes
100
80
60
40
20
0
0
20
40
60
Zeit [min]
Morphin
Morphin + Komponente B

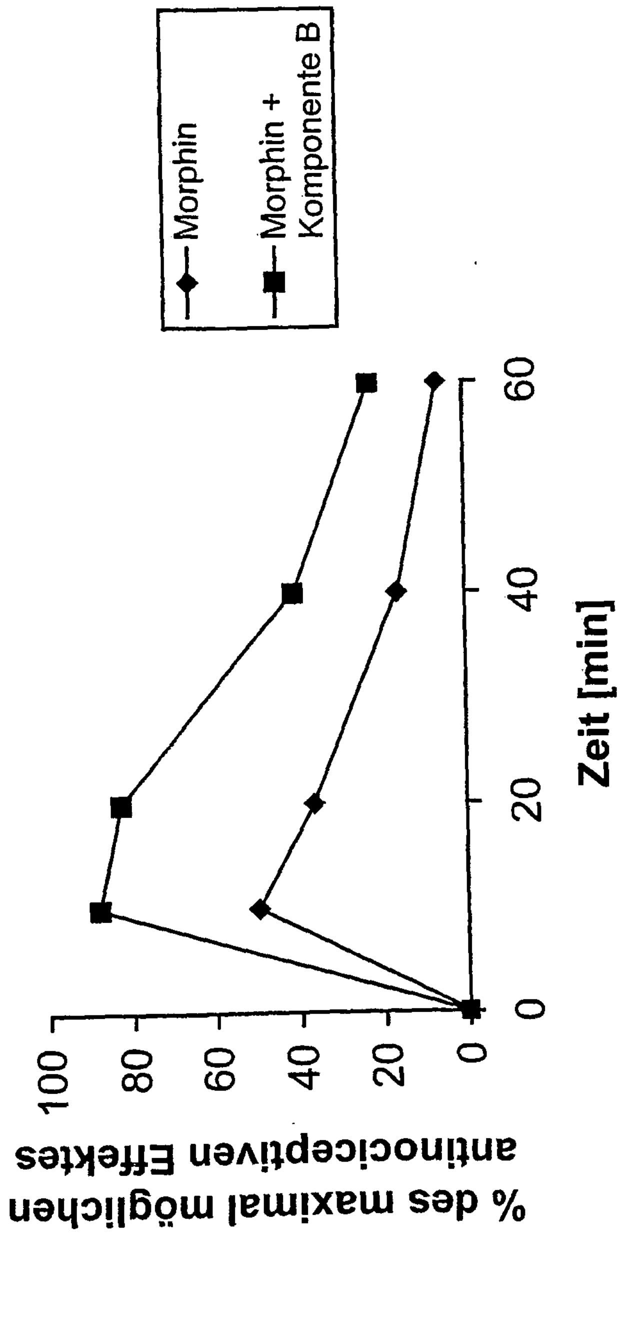
% des maximal möglichen antinociceptiven Effektes
100
80
60
40
20
0
0
20
40
60
Zeit [min]
Morphin
Morphin + Komponente B

Figur 2